(19) Europäisches Patentamt
European Patent Office
Office européen des brevets

(11) **EP 0 906 904 B1**

(12) **EUROPÄISCHE PATENTSCHRIFT**

(45) Veröffentlichungstag und Bekanntmachung des Hinweises auf die Patenterteilung:
**04.09.2002 Patentblatt 2002/36**

(51) Int Cl.[7]: **C07C 303/02**, C07C 309/04

(21) Anmeldenummer: **98118534.1**

(22) Anmeldetag: **30.09.1998**

(54) **Verfahren zur Herstellung von Methansulfonsäure**

Process for the preparation of methanesulfonic acid

Procédé pour la préparation d'acide méthanesulfonique

(84) Benannte Vertragsstaaten:
**AT BE CH DE DK ES FI FR GB GR IE IT LI LU NL PT SE**

(30) Priorität: **04.10.1997 DE 19743901**

(43) Veröffentlichungstag der Anmeldung:
**07.04.1999 Patentblatt 1999/14**

(73) Patentinhaber: **Grillo-Werke AG**
**D-47169 Duisburg (DE)**

(72) Erfinder:
• **Biertümpel, Ingo, Dr.**
**40629 Düsseldorf (DE)**
• **Driemel, Klaus, Dipl.-Ing.**
**47259 Duisburg (DE)**
• **Van de Flierdt, Joachim, Dr.**
**46535 Dinslaken (DE)**
• **Rohe, Dieter M. M., Dr.**
**46537 Dinslaken (DE)**

(74) Vertreter:
**Werner, Hans-Karsten, Dr.Dipl.-Chem. et al**
**Patentanwälte**
**Von Kreisler-Selting-Werner**
**Postfach 10 22 41**
**50462 Köln (DE)**

(56) Entgegenhaltungen:
**CH-A- 101 817        CH-A- 101 818**

• **M. PANTLISCHKO ET AL: MONATSHEFTE FÜR CHEMIE, Bd. 89, 1958, Seiten 285-287, XP002092953**
• **P.K. DUTT: J. CHEM. SOC., Bd. 125, 1924, Seiten 1463-1465, XP002092954**
• **DATABASE CHEMABS CHEMICAL ABSTRACTS SERVICE, COLUMBUS, OHIO, US STN, accession no. 77:33931, XP002092955 & SU 335 240 A (L.V. PISAZZHEVSKII)**

EP 0 906 904 B1

**Beschreibung**

Die vorliegende Erfindung betrifft ein Verfahren zur Herstellung von Methansulfonsäure.

Methansulfonsäure ist eine starke Säure, die korrodierend, aber nicht oxidierend wirkt und als farblose Substanz mit einem Schmelzpunkt von 20°C vorliegt.

Methansulfonsäure findet in der galvanischen Industrie sowie bei der organischen Synthese, insbesondere als Katalysator bei Alkylierungen, Veresterungen und Polymerisationen eine Anwendung. Darüber hinaus dient Methansulfonsäure als Ausgangsprodukt für die Herstellung von Methansulfonylchlorid.

Die Herstellung erfolgt überwiegend durch Oxidation von Methylthiol oder Dimethyldisulfid mit Hilfe von Salpetersäure, Wasserstoffperoxid, Chlor oder unter Einsatz elektrochemischer Verfahren.

Weiterhin bekannt ist die Umsetzung von Natriumsulfit mit Dimethylsulfat; vgl. Ullmanns Encyklopädie der technischen Chemie, Bd. 16 1965, Seiten 567/550.

Ju CH 101 817 wird ein Verfahren zur Darstallung von methans sulfonsaurem Calcium aus einem Methylester der Schwefolsäure und Ammoniumsulfit beschrieben.

CH 10 18 18 betrifft ein Verfahren zur Herstellung von reines, wasser freier Methansulfonsäure aus ihren Metallsalzen mit schwerflüchtiger Säure.

Der Erfindungsbeschreibung zum Urheberschein 335240 der UdSSR, veröffentlicht am 25. Mai 1972, ist ein Verfahren zur Gewinnung von Methansulfonsäure zu entnehmen, bei dem äquimolare Mengen basischer Sulfite mit Dimethylsulfat umgesetzt werden, indem man Dimethylsulfat im Laufe von ca. 20 Minuten in eine gesättigte Lösung eines basischen Sulfits unter Erhitzung der Reaktionsmasse auf 97 bis 99°C gibt, die erhaltene Reaktionsmasse höchstens 35 Minuten stehen läßt, anschließend den Reaktionsansatz mit konzentrierter Schwefelsäure behandelt, einer Vakuumdestillation unterzieht und das Produkt auf bekannte Weise reinigt.

Es ergibt sich dabei folgende Reaktionsgleichung für die Umsetzung des Dimethylsulfats mit Natriumsulfit

$$SO_3{}^{2-}+CH_3O\text{-}SO_2\text{-}OCH_3 \rightarrow CH_3\text{-}SO_3{}^{-}+{}^{-}O\text{-}SO_2\text{-}OCH_3$$

Nachteilig in diesem Verfahren ist, daß nur eine Methylgruppe des Dimethylsulfats genutzt wird und das Nebenprodukt Methylsulfat bei pH-Werten von unter 7 freie Methylschwefelsäure bildet, die im Gleichgewicht mit Schwefelsäure und dem toxikologisch nicht unbedenklichen Dimethylsulfat steht.

Die vorliegende Erfindung hat sich somit zur Aufgabe gestellt, ein Verfahren zur Herstellung von Methansulfonsäure zu entwickeln, das ausgehend von Dimethylsulfat und Natriumsulfit höhere Ausbeuten bezogen auf Dimethylsulfat ergibt und keine Nebenprodukte bildet, die später im Abwasser erneut und unkontrolliert erneut Dimethylsulfat bilden können.

Die erfindungsgemäße Aufgabe wird gelöst durch ein Verfahren mit den Merkmalen des Patentanspruchs 1.

Beim erfindungsgemäßen Verfahren findet eine Umsetzung von Sulfitionen in einem wäßrigen System mit Dimethylsulfat bei erhöhter Temperatur statt, wobei das molare Verhältnis von Sulfitionen zu Dimethylsulfat 1,5 bis 2,5:1, vorzugsweise 2:1 beträgt und der Reaktionsansatz mindestens zwei Stunden, vorzugsweise vier Stunden bei der erhöhten Temperatur gehalten wird und die Methansulfonsäure durch anschließende Umsetzung mit einer starken Säure freigesetzt wird.

Die Reaktionsgleichung des erfindungsgemäßen Verfahrens lautet

$$2\,SO_3{}^{2-}+CH_3O\text{-}SO_2\text{-}OCH_3 \rightarrow 2\,CH_3\text{-}SO_3{}^{-}+SO_4{}^{2-}$$

Das erfindungsgemäße Verfahren erreicht durch die Erhöhung des Verhältnisses zwischen Sulfitionen und Dimethylsulfat und die deutlich verlängerte Reaktionszeit eine Übertragung beider Methylgruppen vom Dimethylsulfat auf die Sulfitionen.

Neben der damit verdoppelten theoretischen Ausbeute, bezogen auf Dimethylsulfat, fällt als Nebenprodukt freies Sulfat an, das in Form seines Salzes, beispielsweise Natriumsulfat, toxikologisch unbedenklich ist.

Im Reaktionsansatz wird der pH-Wert in einem Bereich größer oder gleich 6 gehalten und gegebenenfalls durch Zugabe einer Base wie NaOH auf diesen Bereich eingestellt.

Die Sulfitionen im erfindungsgemäßen Verfahren werten als Natriumsulfit oder Natriumpyrosulfit $Na_2S_2O_5$ zur Verfügung gestellt.

Die bevorzugte Reaktionstemperatur liegt oberhalb von 60°C und noch mehr bevorzugt oberhalb von 95°C, so daß in besonders einfacher Weise die Reaktionsführung durch Erhitzen unter Rückfluß erfolgen kann. Gewünschtenfalls kann die Reaktion auch bei erhöhtem Druck beispielsweise bei 2 bis 5 bar durchgeführt werden. Vorzugsweise wird aber aus Kosten- und Sicherheitsgründen bei Normaldruck oder sogar leichtem Unterdruck gearbeitet.

Anschließend erfolgt im erfindungsgemäßen Verfahren die Umsetzung mit einer starken Säure, beispielsweise Schwefelsäure, und Aufreinigung der Methansulfonsäure, beispielsweise durch Destillation.

Das folgende Beispiel soll das erfindungsgemäße Verfahren erläutern.

In einem 2-l-Dreihalskolben mit Rührer, Innenthermometer, Tropftrichter und Rückflußkühler wird eine Suspension von 300 g (2,38 mol) Natriumsulfit in 1,1 l Wasser auf 95 bis 100°C erhitzt. Bei dieser Temperatur liegt eine klare Lösung vor. Innerhalb von 30 Minuten werden unter Rühren 135 g (1,07 mol) Dimethylsulfat zugetropft. Anschließend wird vier Stunden bei 90 bis 100°C gerührt. Innerhalb dieses Zeitraums wird der pH-Wert der Lösung überwacht. Wenn der pH-Wert unter 6,0 fällt, wird eine 20%ige Natriumsulfitlösung zugetropft, bis der pH-Wert wieder einen Wert von über 7,0 erreicht. Danach wird der Reaktionsansatz mit 450 g konzentrierter $H_2SO_4$ versetzt und vorsichtig bei einem Druck von weniger als 3 kPa destilliert.

Die Wiederholungen der Umsetzung bei Drücken bis 5 bar ergeben ebenfalls gute und hohe Ausbeuten bezogen auf eingesetztes Dimethylsulfat, nämlich 75 bis 85 %.

**Patentansprüche**

1. Verfahren zur Herstellung von Methansulfonsäure durch Umsetzung von Sulfitionen in einem wäßrigen System mit Dimethylsulfat bei erhöhter Temperatur und anschließender Umsetzung mit einer starken Säure, **dadurch gekennzeichnet, daß** das molare Verhältnis von Sulfitionen zu Dimethylsulfat 1,5 bis 2,5:1, vorzugsweise 2:1 beträgt und der Reaktionsansatz mindestens zwei Stunden, vorzugsweise vier Stunden bei der erhöhten Temperatur gehalten wird,
daß der pH-Wert des Reaktionsansatzes durch Zugabe einer Base im Bereich größer oder gleich 6 gehalten wird, und dass
die Sulfitionen in Form von Natriumsulfit oder Natriumpyrosulfit zur Verfügung gestellt werden.

2. Verfahren nach Anspruch 1
**dadurch gekennzeichnet, daß** die Reaktion bei einer Temperatur größer oder gleich 95°C durchgeführt wird.

**Claims**

1. A process for the preparation of methanesulfonic acid by reacting sulfite ions within an aqueous system with dimethyl sulfate at an elevated temperature and adding a strong acid subsequently, **characterized in that** the molar ratio of sulfite ions to dimethyl sulfate is from 1.5 to 2.5:1, preferably 2:1 and the reaction charge is maintained at the elevated temperature for at least two hours, preferably four hours, the pH value of the reaction charge is kept in the range greater than or equal to 6 by adding a base and the sulfite ions are provided in the form of sodium sulfite or sodium pyrosulfite.

2. The process according to claim 1, **characterized in that** the reaction is performed at a temperature greater than or equal to 95 °C.

**Revendications**

1. Procédé de préparation d'acide méthane-sulfonique, par conversion d'ions sulfites en un système aqueux avec du diméthylsulfate, à température augmentée et conversion subséquente avec un acide fort, **caractérisé en ce que** le rapport molaire entre les ions sulfites et le diméthylsulfate est de 1,5 à 2,5:1, de préférence de 2:1, le mélange de réaction étant maintenu au moins deux heures, de préférence 4 heures, à la température augmentée,
**en ce que** la valeur de pH du mélange de réaction est maintenue dans une plage supérieure ou égale à 6, par addition d'une base, et **en ce que**
les ions sulfites sont fournis sous la forme de sulfite de sodium ou pyrosulfite de sodium.

2. Procédé selon la revendication 1, **caractérisé en ce que** la réaction est conduite à une température supérieure ou égale à 95°C.